# EUROPEAN PATENT APPLICATION

(11) **EP 1 576 928 A1**
(43) Date of publication of application: **21.09.2005**
(21) Application number: 04405161.3
(22) Date of filing: 18.03.2004
(51) Int. Cl.: A61B 17/11, A61F 2/06

(54) **Flanged graft for vascular anastomosis and bypass**

(71) Applicant: Enzler, Markus, 8704 Herrliberg (CH)
(72) Inventor: Enzler, Markus, 8704 Herrliberg (CH)
(74) Representative: Störzbach, Michael Andreas

(57) **Abstract**

A flanged graft (100), instruments and methods are provided for forming vascular anastomosis. The flanged graft (100) has a tubular body member (102) and a flange (104). The tubular body member (102) is used as a conduit for fluid between a donor vessel and a recipient vessel. The flange (104) is attached to a donor vessel and atleast one recipient vessel, with the help of the flange and its extensions (110, 112), using a tissue adhesive. A vessel cutter, introduced through the tubular body member (102) of the flanged graft (100), is used to cut a hole in the wall of the donor and the recipient vessel. The flanged graft (100) is thereafter clamped. Two separate flanged grafts (100) attached to the donor and the recipient vessels are joined together to complete the vascular bypass between the donor and the recipient vessel. The method helps in achieving sutureless anastomosis during endoscopic or open vascular bypass surgery without clamping body vessels.

## Description

### BACKGROUND

The present invention relates to a flanged graft suitable for forming vascular anastomoses with body vessels. More particularly, the present invention relates to a flanged graft and method of using the flanged graft for establishing a bypass between body vessels with decreased blood loss, while also minimizing or eliminating the requirement of sutures.

Vascular insufficiency usually occurs as a result of an occlusion or narrowing of the lumen of blood vessel due to formation of atheromatous plaques inside the lumen of the vessel. Vascular insufficiency may lead to tissue ischemia due to decreased blood supply to the region supplied by the affected vessel.

Vascular bypass surgery is one of the frequently used treatment options for the treatment of vascular insufficiency. During vascular bypass surgery, an autologous or a prosthetic graft is anastomosed to a donor vessel and a recipient vessel. The process of establishing an anastomosis, between the graft and either the donor vessel or the recipient vessel, includes making an incision in the vessel wall and attaching the graft to the wall of the donor vessel or the recipient vessel, which is usually done by using sutures.

US patent numbers 6190590, 6248117, 6623494 describe various types of grafts and methods for establishing proximal and distal anastomoses between the graft and the blood vessel. During anastomosis, the graft needs to be attached carefully to prevent fluid leakage at the site of anastomosis. Moreover, the body vessel needs to be clamped proximally and distally during the formation of the anastomosis to prevent massive blood loss. The graft can be attached to the vessel wall using either sutures or various other alternatives available to suturing. These include, amongst others, tissue bonding techniques and the use of bio-adhesives. US patent application no. 5755778 titled, "Anastomosis Device", describes the use of one such graft for establishing anastomosis without the necessity to employ sutures.

For performing vascular bypass surgery involving the aorta, present methods include, amongst other methods, techniques for accessing the aorta through an endoscope and establishing anastomosis between the graft and the aorta. A paper by J. Mack et. al., " Initial experience with proximal anastomosis performed with a mechanical connector", Ann Thorac Surg 2003; 75: 1866-1871, provides details about one such technique. In this technique the thoracic aorta is accessed using a thoracoscope, a hole is cut in the aortic wall using an aortic cutter, and a St. Jude anastomotic stent® is used for establishing anastomosis between the aorta and the graft.

Although several types of grafts and methods exist for establishing anastomosis, they have one or more of the following limitations associated with them. Recent advancements in the surgical techniques and instruments for vascular bypass surgery are directed towards performing effective vascular bypass surgery using very small incisions. However, as the size of the surgical incision required is reduced, it becomes an increasingly difficult and time-consuming procedure to effectively establish anastomosis between the graft and the vessel wall, especially while using sutures.

Increased time to attach the graft to the wall of the vessel may cause increased blood loss in the time interval between making an incision in the wall of the vessel and completion of the anastomosis. To decrease blood loss, the vessel usually needs to be clamped. However, during clamping of the vessel, there is a risk of causing ischemic damage to the organs, or even paralysis of the tissues supplied by the vessel, due to the interruption of blood supply. Moreover, clamping of calcified vessels can damage the vessel wall by crushing calcified plaques that may embolize into the periphery or compromise locally the arterial lumen.

Hence, there exists a need for an effective graft that can be used for establishing anastomosis with decreased blood loss, without the need for clamping the vessel. Moreover, the graft and the method for forming vascular anastomosis should minimize or eliminate the requirement of sutures. Furthermore, the system and method should be simple to use.

### SUMMARY

An object of the present invention is to provide a flanged graft to be used for establishing vascular anastomosis.

Another object of the present invention is to provide a graft system, using the flanged graft and a centering bolt, for establishing vascular anastomosis.

Yet another object of the present invention is to provide a graft system, using the flanged graft and a centering bolt, for achieving clampless and sutureless vascular anastomosis.

Yet another object of the present invention is to provide a vessel cutter for cutting holes in the vessel wall, the diameter of the holes being almost equal to the internal diameter of a tubular body member of the flanged graft.

Yet another object of the present invention is to provide a method for using the graft system along with the vessel cutter for performing vascular bypass surgery, wherein the method helps in achieving sutureless and clampless anastomosis. The method further helps in minimizing blood loss during anastomosis.

According to one embodiment of the present invention, a graft system and a vessel cutter are provided for use in vascular bypass surgery. The graft system comprises a flanged graft and a centering bolt. The flanged graft comprises a tubular body member having a central lumen, and a flange. The central lumen acts as a conduit for fluid between a donor and a recipient vessel. The flange has extensions, and can be tailored according to the width of the vessel. The flange and its extensions help in attaching the flanged graft to the vessel by encircling the extensions around the wall of the vessel. The centering bolt is placed inside the central lumen and helps in centering the vessel cutter inside the central lumen.

According to one embodiment of the present invention, the vessel cutter consists of three co-axial structures: an inner centering rod, a middle component and an outer cutting cylinder. The inner centering rod is used to center the vessel cutter inside the central lumen of the flanged graft. The centering rod has a corkscrew. The corkscrew helps to hook on to the segment of the vessel wall to be cut, along with the cut part of the flange and the centering bolt, and thus prevents them from embolizing in to the periphery. The outer cutting cylinder has a cutting blade. The cutting blade is used to cut holes in the wall of the vessel. The middle component helps in centering the corkscrew inside the centering bolt. The middle component also helps in centering the instrument in the lumen of the graft by fitting it around the centering bolt and preventing damage to the walls of the flanged graft from the cutting blade of the outer cutting cylinder.

According to one embodiment of the present invention, a method is presented for establishing anastomosis between the graft system and a donor vessel or a recipient vessel. Flanged grafts are attached to the donor vessel and the recipient vessel. The attachment is secured with the help of flange extensions, which completely encircle the wall of the vessel. Means to attach the flange extensions to the wall of the vessel may include using glue, staplers, sutures, Velcro, hooks or combinations thereof. This helps in achieving an expedient, but durable and watertight sutureless anastomosis without the need for clamping the body vessel. After the flanged graft has been attached to the wall of the vessel, the vessel cutter is introduced through the central lumen of the flanged graft, and is used to cut a hole in the wall of the vessel. Thereafter, the vessel cutter is withdrawn, and in order to prevent blood loss the tubular body member is clamped. Finally, the flanged grafts attached to the donor and the recipient vessels are connected together to complete the vascular bypass between the donor and the recipient vessel. The two grafts can be connected using sutures or a connecting graft.

Additional objects and advantages of the invention will become apparent to those skilled in the art upon reference to the detailed description taken in conjunction with the provided figures.

### BRIEF DESCRIPTION OF THE DRAWINGS

The preferred embodiments of the invention will hereinafter be described in conjunction with the appended drawings provided to illustrate and not to limit the invention, wherein like designations denote like elements, and in which:
FIG. 1 is a side view of a flanged graft 100, which is used for forming anastomosis during vascular bypass surgery, in accordance with one embodiment of the present invention.
FIG. 2 is a side view of a type of flanged graft 200, which is used for forming anastomosis during vascular bypass between one donor vessel and two recipient vessels, in accordance with one embodiment of the present invention;
FIG. 3 A is a side view of a graft system 300, which consists of a flanged graft and a centering bolt, in accordance with one embodiment of the present invention;
FIG. 3 B is a side view of the centering bolt, in accordance with one embodiment of the present invention;
FIG. 4 A is a representation of the vessel cutter 400, in accordance with one embodiment of the present invention;
FIG. 4 B is a representation of a variation of the vessel cutter 400, in accordance with another embodiment of the present invention;
FIG. 5 is an illustration of a completed vascular bypass procedure, showing anastomoses between the flanged graft and thoracic aorta, and between the flanged graft and the common iliac artery. The two flanged grafts connected by a connecting graft are shown
FIG. 6 is an illustration of the use of a type of flanged graft for performing vascular bypass surgery between thoracic aorta and common iliac artery on one side and common femoral vessels on the other side.
FIG. 7 is an illustration of the completed anastomosis between flanged graft 100 and a body vessel.
FIG. 8 is a flowchart illustrating the method for performing vascular bypass between thoracic aorta and the common iliac artery using the graph system;
FIG. 9 is a flowchart illustrating the method for attaching the graft system to the thoracic aorta or the common iliac artery; and
FIG. 10 is a flowchart illustrating the method for completing the anastomosis between graft system and the thoracic aorta or the common iliac artery.

### DESCRIPTION OF PREFERRED EMBODIMENTS

The present invention discloses a flanged graft and a method for forming vascular anastomosis. The flanged graft is used for establishing anastomosis with a donor vessel and another flanged graft is used for establishing anastomosis with a recipient vessel. The flanged grafts anastomosed to the donor and the recipient vessels are joined together for completing the vascular bypass between the donor vessel and the recipient vessel.

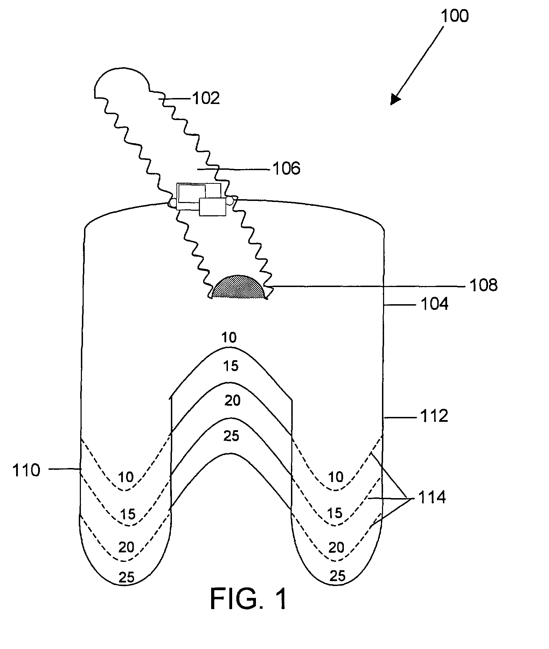

FIG. 1 is a side view of a flanged graft, which is used for forming anastomosis during vascular bypass surgery, in accordance with one embodiment of the present invention. The flanged graft is hereafter referred to as graft 100. Graft 100 comprises a tubular body member 102 and a flange 104. Tubular body member 102 has a central lumen 106. Central lumen 106 is used as a conduit for fluid between the donor vessel and the recipient vessel.

Graft 100 has a flanged end and a free connecting end. The flanged end of graft 100 is the end that is used for establishing anastomosis between graft 100 and the donor vessel or the recipient vessel. The free connecting end of graft 100 is used to connect graft 100 to the free connecting end another graft 100 to complete the vascular bypass between the donor and atleast one recipient vessel.

In accordance with one embodiment of the present invention, the free connecting ends of two separate graft 100 can be connected together using a connecting graft. The connecting graft, in accordance with one embodiment of the present invention, is designed in the shape of a tubular body member 102 to join the free connecting ends of the two separate graft 100.

Flange 104 is attached to the flanged end of tubular body member 102. Flange 104 is wrapped around the wall of the donor or the recipient vessel. Flange 104 helps in attaching graft 100 to the donor vessel or the recipient vessel with the help of a tissue adhesive or sutures.

Flange 104 has a flange membrane 108. Flange membrane 108 is continuous with flange 104 and enters central lumen 106. Flange membrane 108 helps during the cutting of the vessel wall by helping to position the cutting device inside central lumen 106, with the help of a centering device.

Flange 104 has extensions 110 and 112. Length of extensions 110 and 112 can be adjusted according to the diameter of the donor vessel or the recipient vessel. Extensions 112 and 110 have markings 114 on them to cut their length according to the diameter of the vessel. Markings 114 can be used to cut the length of extensions 112 and 114 to 10, 15, 20 or 25 millimeters depending on the diameter of the donor or recipient vessel.

The Extensions 110 and 112, along with flange 104 help in attaching graft 100 to the donor vessel and the recipient vessel wall. Flange 104 and extensions 110 and 112 can be attached to the wall of the vessel by means like sutures, staplers, hooks, tissue bonding techniques and tissue adhesives or combinations thereof.

In accordance with one embodiment of the present invention, flange 104 and extensions 110 and 112 are attached to the wall of the vessel using tissue adhesives. In accordance with to one embodiment of the present invention, the tissue adhesive is Bioglue®.

Extensions 110 and 112 along with flange 104 tightly encircle the wall of the donor vessel and the recipient vessel. This helps in providing durable and leak less anastomosis by firmly attaching graft 100 around the vessel wall.

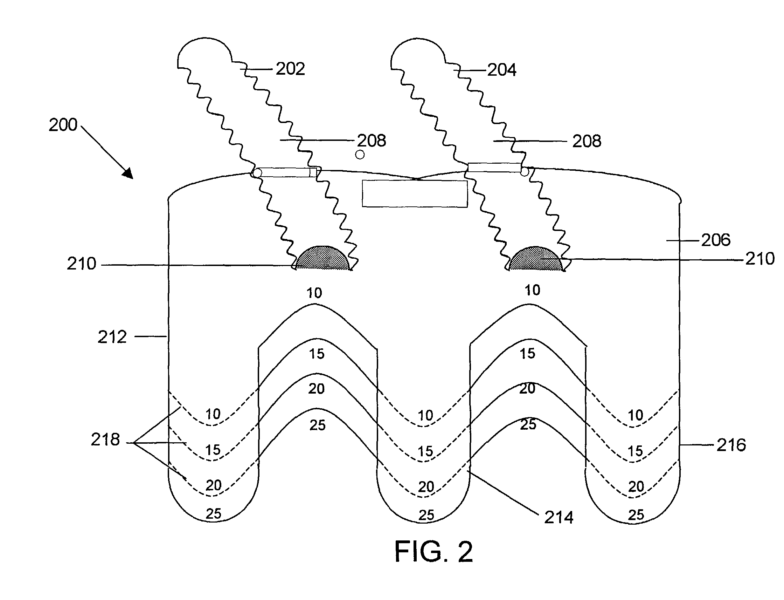

In accordance with one embodiment of the present invention, graft 100, as described above, can be used for forming vascular bypass between one donor and one recipient vessel. In case vascular bypass needs to be formed between one donor and more than one recipient vessels, graft 100 needs to be modified. One such modification of graft 100, herein called graft 200, is shown in FIG. 2. In accordance with one embodiment of the present invention, graft 200 is used for forming vascular bypass between one donor vessel and two recipient vessels.

Graft 200 comprises tubular body members 202 and 204, and flange 206. Tubular body members 202 and 204 have a central lumen 208. Each central lumen 208 is used as a conduit for fluid between the donor vessel and two separate recipient vessels. Tubular body members 202 and 204 have a flanged end and a free connecting end.

The flanged end of tubular body members 202 and 204 is the end that is used for establishing anastomosis between graft 200 and the wall of the donor vessel. The free connecting ends of tubular body members 202 and 204 are used to connect each tubular body member 202 and 204 either directly to recipient vessels or to two separate graft 100, attached to two recipient vessels, to complete the vascular bypass between the donor and two recipient vessels.

In accordance with one embodiment of the present invention, tubular body members 202 and 204 help in forming aorto-biiliac bypass between the aorta and the two common iliac arteries. Aorto-biiliac bypass may be needed in cases where there is a vascular obstruction involving the bifurcation of the abdominal aorta, and/or the bifurcation of common iliac arteries. In such cases, vascular bypass can be performed between the aorta and the common iliac arteries, external iliac arteries or the femoral arteries using a single graft 200, which joins the aorta and any of the two above mentioned recipient vessels, the aorta acting as the donor vessel, and the common iliac arteries, external iliac arteries or the femoral arteries acting as recipient vessels.

Flange 206 is attached to the flanged end of tubular body members 202 and 204. Flange 206 has flange membrane 210. Flange membrane 210 is continuous with flange 206 and enters central lumen 208 of tubular body members 202 and 204. Flange membrane 210 helps during the cutting of the vessel wall by helping to position the cutting device inside central lumen 208 with the help of a centering device.

Flange 206 has extensions 212, 214 and 216. Length of extensions 212, 214 and 216 can be adjusted according to the diameter of the donor vessel. Extensions 212, 214 and 216 have markings 218 on them to cut their length according to the diameter of the vessel. Markings 218 can be used to cut the length of extensions 212, 214 and 216 to 10, 15, 20 or 25 millimeters depending on the diameter of the donor or recipient vessel.

Extensions 212 and 214 along with flange 206 help in attaching graft 200 to the wall of the donor vessel. Flange 206 and extensions 212 and 214 can be attached to the wall of the vessel by means like sutures, staplers, hooks, tissue bonding techniques and tissue adhesives or combinations thereof.

In accordance with one embodiment of the present invention, flange 206 and extensions 212 and 214 are attached to the wall of the donor vessel using tissue adhesives. According to one embodiment of the present invention, the tissue adhesive is Bioglue®.

Extensions 212 and 214 along with flange 206 tightly encircle the wall of the donor vessel. This helps in providing durable and leak less anastomosis by firmly attaching graft 200 to the wall of the donor vessel.

In accordance with one embodiment of the present invention, tubular body member 102, flange 104 and flange membrane 108 are made of Dacron. In accordance with another embodiment of the present invention, tubular body member 102, flange 104 and flange membrane 108 are made of polytetrafluoroethylene (PTFE).

After graft 100 is attached to the donor vessel or the recipient vessel wall, a hole needs to be cut in the wall of the donor or the recipient vessel. This can be done by using a cutting device or a punching device, which is introduced through central lumen 106 of graft 100, to cut or punch a hole in the wall of the vessel. However, when a punching or a cutting device is introduced inside central lumen 106, there is a risk of damage being caused to the walls of tubular body member 104 from the cutting device. To prevent this, a centering bolt is placed inside central lumen 106.

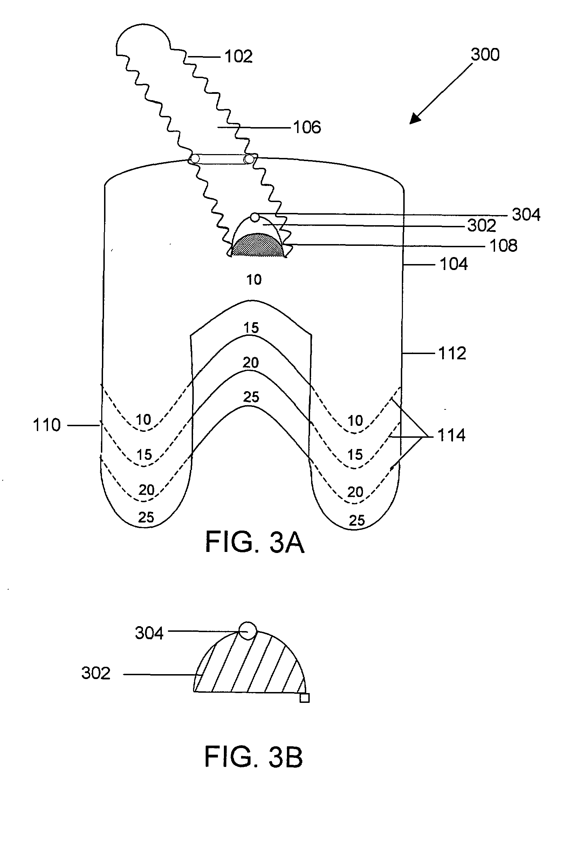

FIG. 3 A shows one such arrangement, in accordance with one embodiment of the present invention, wherein a centering bolt 302 is placed inside central lumen 106 of graft 100. Graft 100 along with centering bolt 302 is hereafter called graft system 300.

FIG. 3 B shows centering bolt 302, in accordance with one embodiment of the present invention. Centering bolt 302 has a central boring 304. Centering bolt 302 facilitates in centering a cutting device and in preventing damage to the walls of the tubular body member from the cutting device.

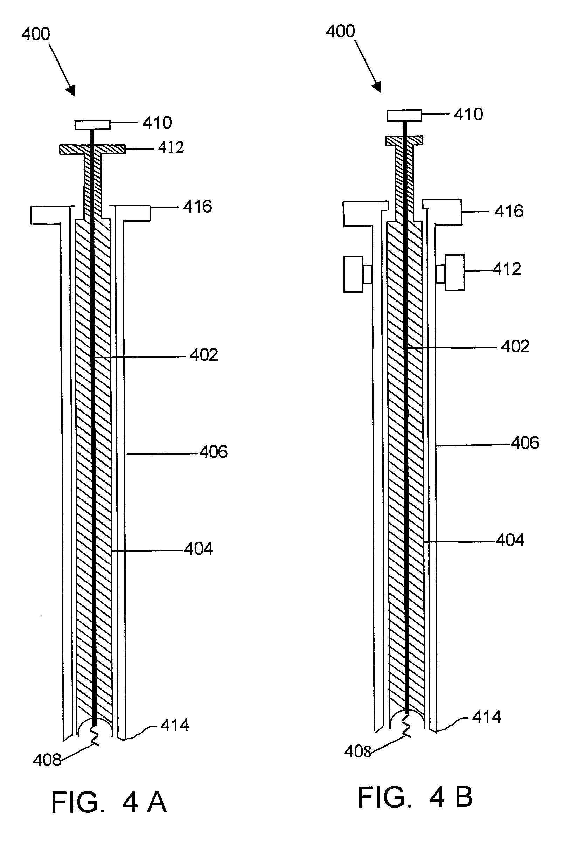

FIG. 4A is a view of the vessel cutter 400, in accordance with one embodiment of the present invention. Vessel cutter 400 is used to cut holes in the vessel wall. Vessel cutter 400 comprises three coaxial structures, inner centering rod 402, middle component 404 and outer cutting cylinder 406. Vessel cutter 400 helps in cutting a hole in the wall of the vessel.

Inner centering rod 402 has corkscrew 408 at the front end of vessel cutter 400 and a thumbwheel 410 at the rear end of vessel cutter 400. Front end of vessel cutter 400 is the end that is away from the surgeon, while using vessel cutter 400 to cut a hole in the wall of the vessel. Rear end of vessel cutter 400 is the end that is towards the surgeon, while using vessel cutter 400. Corkscrew 408 is used to prevent the cut wall of the vessel and the centering bolt from embolizing through the lumen of the donor body vessel and the recipient body vessel into the periphery. Thumbwheel 410 is used to turn the corkscrew 408.

Middle component 404 has a grab handle 412 at the rear end of vessel cutter 400. Middle component 404 is used to balance and hold vessel cutter 400 while cutting a hole in the wall of the vessel. Middle component 404 has a recess near the front end of vessel cutter 400 to help it fit properly around centering bolt 210. Middle component 404 helps to center corkscrew 408 inside central boring 304 of central bolt 302.

Outer cutting cylinder 406 has a cutting blade 414 at the front end of vessel cutter 400 and a rotating handle 416 at the rear end of vessel cutter 400. In accordance with one embodiment of the present invention, cutting blade 414 has serrated edges to cut a hole in the wall of the vessel. In accordance with another embodiment of the present invention, the edges of cutting blade 414 are gemmed to help in cutting a hole in the wall of the vessel; and to help in breaking any calcifications or atheromatous plaque that may be present at the site of the planned anastomosis.

Rotating handle 416 is firmly attached to outer cutting cylinder 406 at the rear end of vessel cutter 400. Rotating handle 416 is used to rotate cutting blade 414 for cutting the vessel wall.

In accordance with one embodiment of the present invention, the grab handle may be located on the outer cylinder of vessel cutter. FIG. 4B shows one such vessel cutter, wherein grab handle 412 is attached to outer cutting cylinder 406 via a roller or needle bearing. Grab handle 412 helps to stabilize the vessel cutter using one hand, while the surgeon is using the other hand for rotating cutting blade 414 using rotating handle 416.

Vessel cutter 400 should be long enough to allow access to the aorta in all patients. The diameter of vessel cutter 400 should be less than the diameter of central lumen 106 to allow easy introduction of vessel cutter 400 inside central lumen 106. In accordance with one embodiment of the present invention, the shaft length of vessel cutter 400 is 25 cm. This helps in accessing the aorta even in fat patients. The outer diameter of vessel cutter 400 is 9.5 mm. This is less than the diameter of central lumen 106 of graft 100. This helps in accommodating up to 50 cm length of graft 100 over a vessel cutter 400 of 25cm length. Further, corkscrew 408 has a diameter of 2 mm, to pass through central boring 212 of centering bolt 210. Also, in idle position, cutting blade 414 is slightly short of length as compared to middle component 404. This helps in preventing damage to tubular body element 102.

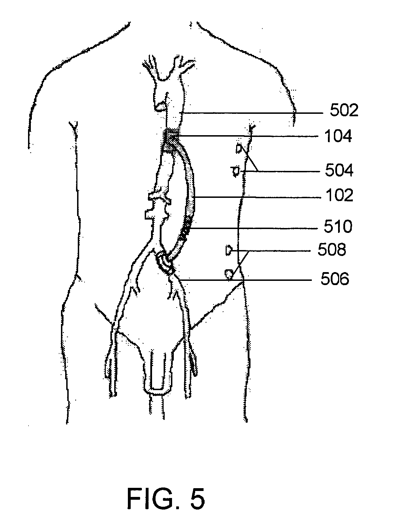

FIG. 5 is an illustration of a completed vascular bypass procedure, in accordance with one embodiment of the present invention. FIG. 5 shows anastomoses between graft 100 and TA 502 and graft 100 and CIA 506. The two grafts 100 are shown to be connected by a connecting graft 510. TA 502 is accessed via thoracoscopic ports 504 and CIA 506 is accessed through retroperitoneal ports 508.

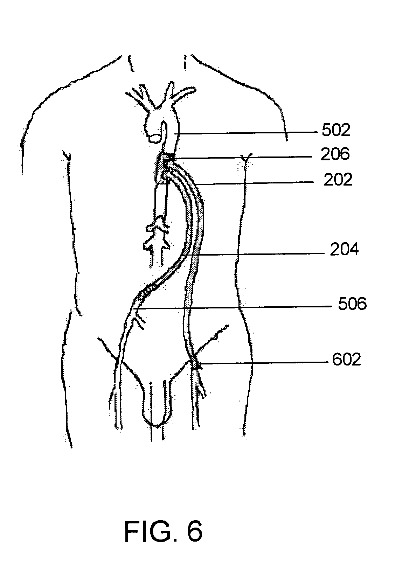

FIG. 6 is an illustration of the use of graft 200, in accordance with one embodiment of the present invention, for forming anastomosis between TA 502 and CIA 506 on one side and common femoral artery 602 on the other side. The anastomosis with CIA is performed using a connecting graft. The anastomosis with common femoral artery is performed by directly attaching free connecting end of tubular body member 202 with the wall of common femoral artery.

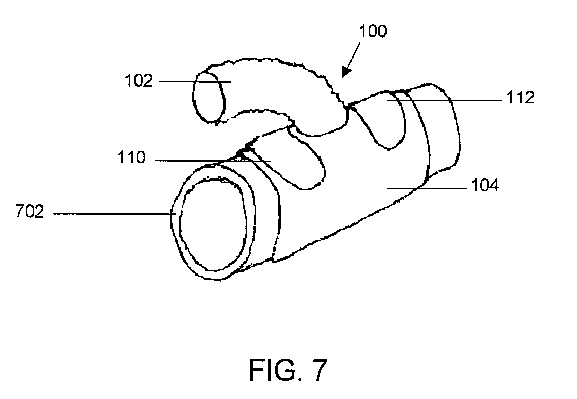

FIG.7 is an illustration of the completed anastomosis between flanged graft 100 and a body vessel 702, in accordance with one embodiment of the present invention. It shows the use of extensions 112 and 114 to help attach flanged graft 100 to body vessel 702. Also shown are tubular body member 102 and flange 104.

Having disclosed graft 100, graft system 300 and vessel cutter 400, a method for forming vascular anastomosis using these is disclosed hereinafter. As an example of forming anastomosis during vascular bypass surgery, a method for performing bypass between the thoracic (TA) and common iliac arteries (CIA) is described. It will be apparent to one skilled in the art that similar to aorto-iliac bypass, the bypass may involve any two vessels or even more than two vessels, as in the case of aorto-biiliac bypass.

The choice of the site of anastomosis and the recipient vessel depends on the site of the vascular obstruction. In case the vascular obstruction is in the common iliac arteries or is more distal, the most proximal patent vessel is used as a recipient vessel. In accordance with one embodiment of the present invention, the recipient vessel is the external iliac artery. In accordance with another embodiment of the present invention, the recipient vessel is the common femoral artery.

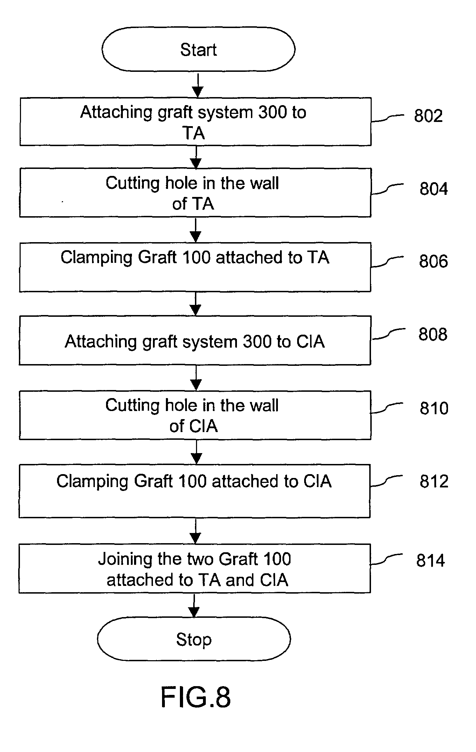

FIG. 8 is a flow chart illustrating the method for performing aorto-iliac bypass using graft system 300, in accordance with one embodiment of the present invention. The method comprises 7 main steps as described in the FIG. 8. At step 802, one graft system 300 is attached to TA. Subsequently, at step 804, a hole is cut in the wall of the TA, through tubular body member 102 of graft system 300. Thereafter, at step 806, tubular body member 102 is clamped with a clamping device to prevent blood loss.

Further, at step 808, a separate graft system 300 is attached to CIA. Subsequently, at step 810, a hole is cut in the wall of CIA. Thereafter, at step 812, tubular body member 102 of graft 100 attached to CIA is clamped.

After cutting a hole in the wall of the vessel, a vascular stent may be used for preventing dissection of the wall of TA and CIA, and for preventing shrinking and/or closure of the hole. Lastly, at step 814, the free connecting ends of the two separate graft 100 of each graft system 300, attached to the TA and the CIA, are joined together to complete the aorto-iliac bypass. Each of the above mentioned steps are discussed in further detail hereinafter.

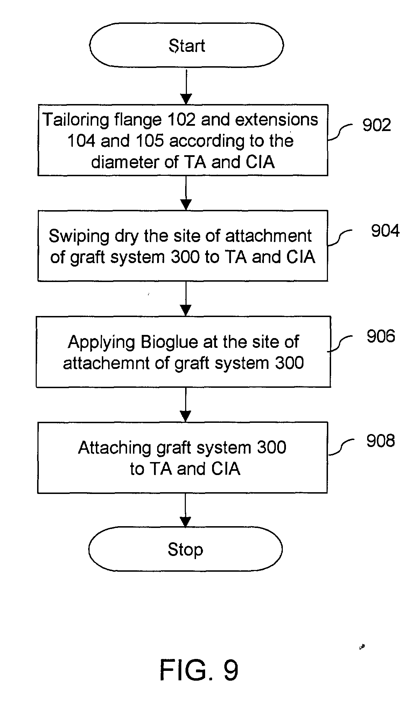

FIG. 9 is a flow chart illustrating the method for attaching graft system 300 to the wall of TA or CIA, in accordance with one embodiment of the present invention. Before step 902 is performed for attaching graft system 300 to the wall of TA and CIA, both the vessels are approached endocopically, using techniques known in the art. At step 902, flange 104 and extensions 110 and 112 of two separate graft systems 300 are tailored according to the diameter of TA and CIA. Further, at step 904, the site of attachment of graft system 300 to TA and CIA is determined and swiped dry. At step 906, a tissue adhesive is applied over the dried site of the TA and CIA and also over flange 104 and extensions 110 and 112.

In accordance with one embodiment of the present invention, the tissue adhesive used is Bioglue®. It will be apparent to one skilled in the art that graft system 300 can also be attached to TA and CIA using other types of tissue adhesives and tissue bonding techniques.

Subsequently, at step 908, one of the tailored graft system 300 is attached to the TA, and the second tailored graft system 300 is attached to CIA. For this, flange 104 and extensions 110 and 112 are encircled around the vessel wall. This helps in achieving leakless anastomosis. Further, stay sutures may be put in place between flange 104 and vessel wall or between the extensions and the flange to completely secure graft system 300. Till step 908, no hole has been cut in the wall of either TA or CIA, so there is no blood loss from the vessels. Also, this method is quick to perform and the process is simple to carry out.

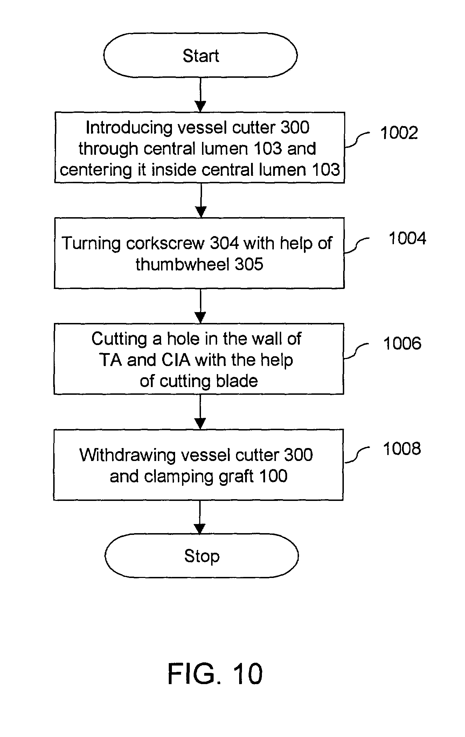

FIG. 10 is a flow chart illustrating the method for completing anastomosis between graft system 300 and TA or CIA using vessel cutter 400. After graft system 300 has been firmly attached to TA or CIA, at step 1002, vessel cutter 400 is introduced through central lumen 308. Vessel cutter 400 is centered inside central lumen 308 with the help of centering bolt 302. The recess in middle component 404, at its front end, fits around centering bolt 302. This helps in positioning vessel cutter 400 properly inside central lumen 308. This also helps in centering corkscrew 408 inside central boring 304.This also helps in preventing damage to the walls of tubular body member 302.

At step 1004, after vessel cutter 400 has been centered inside central lumen 106, and corkscrew 408 is turned with the help of thumbwheel 412. Turning helps corkscrew 408 to penetrate centering bolt 302 through central bore 304, flange membrane 108 and the wall of TA or CIA. Depth of penetration inside the vessel wall is limited by the design of the instrument (to less than 10mm) in order to prevent damage to or penetration of the opposite wall of the artery. The corkscrew helps in hooking the cut wall of the vessel and centering bolt 302 and prevents them from embolizing through the lumen of TA or CIA into the periphery.

Subsequently at step 1006, cutting blade 414 is moved back and forth with the help of rotating wheel 418 to cut through the wall of TA or CIA. A hole is cut in the wall of TA or CIA. Thereafter, at step 1008, vessel cutter 400 is withdrawn from tubular body member 302. A clamp is applied, using clamping instruments known in the art, to prevent blood loss through graft system 300. This completes anastomosis between graft system 300 and TA or CIA.

This method helps in preventing blood loss from the TA or CIA and also eliminates the need to clamp the TA or CIA. Thus, this method helps in achieving anastomosis without the need of clamping arteries. Also, since this method does not necessarily involve the use of sutures, it helps in achieving sutureless anastomosis. This decreases the time needed to form anastomosis and makes the method convenient to use.

To complete the bypass between TA and CIA, the two graft systems 300 attached to TA and CIA need to be connected together. Free connecting ends of both grafts 100 are shortened to appropriate lengths. Using conventional sutures known in the art, the free connecting ends of the two grafts 100 can be joined together.

In accordance with one embodiment of the present invention, the free connecting ends can be joined together by using a connecting graft 510. A dilation balloon is inserted via a puncture into the tubular body member 102 of one of the graft 100 and positioned such as to stick out halfway from the free end. A connecting graft 510 is pulled over the free connecting end of the second graft 100. The free connecting end of second graft 100 is joined with the free connecting end of the first graft 100 over the balloon. Further, tissue adhesive is applied over the joined connecting ends of the two grafts 100. The connecting graft 100 is then pulled over the joined ends. Thereafter, the balloon is inflated. When the adhesive has set, the balloon is then deflated and withdrawn. The puncture site through which the balloon is introduced is sealed using a suture or tissue adhesive alone, or in connection with an extension of the connecting graft 100. Clamps are removed from both graft systems 300 (attached to the TA and CIA) to let the blood flow through the bypass between TA and CIA.

In accordance with one embodiment of the present invention, the free connecting end of graft 100 attached to the TA is directly sutured to the recipient vessel. This helps to avoid the use of two grafts 100, and also simplifies the procedure of vascular bypass surgery.

While the preferred embodiments of the present invention have been illustrated and described, it will be clear that the invention is not limited to these embodiments only. Numerous modifications, changes, variations, substitutions and equivalents will be apparent to those skilled in the art without departing from the spirit and scope of the present invention, as described in the claims.

## Claims

1. A flanged graft (100) suitable for forming a graft between a donor body vessel and a recipient body vessel, the flanged graft comprising:
a.at least one tubular body member (102) acting as a conduit for fluid between a donor body vessel and a recipient body vessel, the tubular body member having a central lumen (106), a fixed end and a connecting end; and
b.a flange (104), the flange helping in attaching the flanged graft at the site of anastomosis, the flange being attached to the fixed end of the tubular body member.

2. The flanged graft of claim 1, **characterized in that** the flange has extensions (110, 112) to encircle around the donor body vessel and the recipient body vessel, the extensions helping in firm attachment of the flanged graft to the donor body vessel and the recipient body vessel and in achieving leak less anastomosis.

3. The flanged graft of claim 1, **characterized in that** the size of the flange can be tailored according to the diameter of the donor body vessel and the recipient body vessel.

4. The flanged graft of claim 1, **characterized in that** a membrane (108) is attached to the flange, the membrane entering into the central lumen of the tubular body member at its fixed end, the membrane helping during the cutting of the body vessel wall, by helping in holding a centering device in place.

5. The flanged graft of claim 1, **characterized in that** the flanged graft is made of a biocompatible material, the biocompatible material being of a kind that can be used safely for forming grafts inside the human body.

6. A graft system (300) suitable for forming a graft between a donor body vessel and a recipient body vessel, the graft system comprising:
a.a flanged graft, the flanged graft comprising:
i.at least one tubular body member (102), the tubular body member acting as a conduit for fluid between a donor body vessel and a recipient body vessel, the tubular body member having a central lumen (106), a fixed end and a connecting end; and
ii. a flange (104), the flange helping in attaching the flanged graft at the site of anastomosis, the flange being attached to the fixed end of the tubular body member.
b.a centering bolt (302) having a central boring (304), the centering bolt being placed inside the central lumen of the tubular body member.

7. The graft system of claim 6, **characterized in that** the flange has extensions (110, 112) to encircle the donor body vessel and the recipient body vessel, the extensions helping in firm attachment of the flanged graft to the donor body vessel and the recipient body vessel, and in achieving leak less anastomosis.

8. The graft system of claim 6, **characterized in that** the size of the flange can be tailored according to the diameter of the donor body vessel and the recipient body vessel.

9. The graft system of claim 6, **characterized in that** a membrane (108) is attached to the flange, the membrane entering into the central lumen of the tubular body member at its fixed end, the membrane helping in holding the centering bolt.

10. The graft system of claim 6, **characterized in that** the flanged graft is made of a biocompatible material, the biocompatible material being of a kind that can be used safely for forming grafts inside the human body.

11. The graft system of claim 6, **characterized in that** the centering bolt is held in place by the membrane, the centering bolt facilitating in cutting the body vessel wall using a cutting device and in preventing damage to the walls of the tubular body member from the cutting device.

12. The graft system of claim 6, further comprising a vessel cutter (400) for cutting holes in the wall of the donor body vessel and the recipient body vessel, the vessel cutter being introduced through the central lumen of the tubular body member.

13. The vessel cutter of claim 12, **characterized in that** the vessel cutter is in the form of coaxial structures, the coaxial structures comprising:
a. an innermost central rod (402) with a front end and a rear end, the central rod comprising:
i.a corkscrew (408) at the front end of the central rod, the corkscrew helping in preventing the cut wall of the vessel and the centering bolt from embolising through the lumen of the donor body vessel and the recipient body vessel into the periphery;
ii. a thumb wheel (410) at the rear end of the central rod, the thumb wheel helping in rotating the corkscrew;
b.a middle component (404), the middle component helping in centering the corkscrew inside the centering bolt, the middle component having a grab handle (412) for supporting the vessel cutter; and
c.an outer cutting cylinder (406) with a front end and a rear end, the outer cutting cylinder helping in cutting holes in the wall of the donor body vessel and the recipient body vessel.

14. The vessel cutter of claim 13, **characterized in that** the outer cutting cylinder comprises:
a.a cutting blade (414) at the front end of the cylinder, the cutting blade being used to cut through the wall of the body vessel; and
b.a rotary handle (416) at the rear end of the cutting cylinder, the rotary handle firmly attached to the cutting cylinder, the rotary handle helping in movement of the cutting blade.

15. The outer cutting cylinder of claim 14, **characterized in that** the cutting blade has at least one of serrated or gemmed edges.

16. A graft system (300) suitable for forming a graft between a donor body vessel and a recipient body vessel, the graft system comprising:
a. a flanged graft, the flanged graft comprising:
i.at least one tubular body member (102), the tubular body member acting as a conduit for fluid between a donor body vessel and a recipient body vessel, the tubular body member having a central lumen (106), a fixed end and a connecting end;
ii. a flange (104), the flange helping to attach the flanged graft at the site of anastomosis, the flange being attached to the fixed end of the tubular body member;
iii. flange extensions (110, 112), the flange extensions helping in firm attachment of the flanged graft to the donor body vessel and the recipient body vessel and in achieving leak less anastomosis;
iv. flange membrane (108), the flange membrane attached to the flange and entering into the central lumen of the tubular body member and helping in holding a centering device;
b.a centering bolt (302), the centering bolt being held inside the central lumen of the tubular body member with the help of the flange membrane, the centering bolt having a central boring (304) and the centering bolt facilitating in cutting the body vessel wall using a cutting device and in preventing damage to the walls of the tubular body member from the cutting device; and
c.a vessel cutter (400), the vessel cutter being in the form of coaxial structures, the coaxial structures comprising:
i.an innermost central rod (402), the central rod having a corkscrew (408) at the front end of the central rod, the corkscrew helping in preventing the cut wall of the vessel and the centering bolt from embolising through the lumen of the donor body vessel and the recipient body vessel into the periphery, and a thumb wheel (410) at the rear end of the central rod, the thumb wheel helping in rotating the corkscrew;
ii. a middle component (404), the middle component helping in centering the corkscrew inside the tubular body member, the middle component having a grab handle (412) for supporting the vessel cutter;
iii. an outer cutting cylinder (406), the cutting cylinder having a cutting blade (414) at the front end of the cylinder, to cut through the wall of the body vessel, and a rotary handle (416) at the rear end of the cutting cylinder, the rotary handle firmly attached to the cutting cylinder, the rotary handle helping in movement of the cutting blade.

17. A bypass method suitable for forming a graft with decreased blood loss between body vessels, the bypass being achieved using a flanged graft, the flanged graft comprising a tubular body member and a flange, the flange having extensions, the method comprising the steps of:
a.attaching the flanged grafts to a donor body vessel and at least one recipient body vessel;
b.cutting a hole in the wall of the donor body vessel and the recipient body vessel through the tubular body member of the flanged graft;
c.introducing a vascular stent for preventing dissection of the wall of the donor body vessel and recipient body vessel and for preventing shrinking and/or closure of the hole;
d.clamping the flanged grafts with a clamping device to prevent blood loss;
e.joining the flanged grafts attached to the donor body vessel and the recipient body vessel to complete the bypass between the body vessels.

18. The method of claim 17, **characterized in that** the step of attaching the flanged grafts to a donor body vessel and at least one recipient body vessel comprises the steps of:
a.approaching and isolating the donor body vessel and the recipient body vessel and drying the site of anastomosis;
b.applying a bio-adhesive at the site of anastomosis; and
c.attaching the flanged grafts at the site of anastomosis by wrapping the flange with its extensions around the wall of the donor body vessel and the recipient body vessel.

19. The method of claim 17, **characterized in that** the donor body vessel and the recipient body vessel are approached endoscopically.

20. The method of claim 17, **characterized in that** the donor body vessel is the thoracic aorta.

21. The method of claim 17, **characterized in that** the recipient body vessel is at least one common iliac artery.

22. The method of claim 17, **characterized in that** the step of cutting a hole in the wall of the donor body vessel and the recipient body vessel through the tubular body member of the flanged graft comprises the steps of:
a.centering a cutting device inside the tubular body member of the flanged graft;
b.using the cutting device for cutting a hole through the wall of the donor body vessel and recipient body vessel; and
c.using a corkscrew type device or a suction device to help in preventing the excised wall of the donor body vessel and recipient body vessel from entering into the lumen of the body vessel and embolizing into the periphery.
